# FASCICULE DE BREVET EUROPEEN

(11) **EP 2 512 414 B1**
(45) Date de publication et mention de la délivrance du brevet: **11.10.2017**
(21) Numéro de dépôt: 10805801.7
(22) Date de dépôt: 14.12.2010
(51) Int. Cl.: A61K 8/34, A61K 8/46, A61K 8/86, A61Q 5/02, A61Q 5/12, A61K 8/36, A61K 8/37, A61Q 5/00

(54) **COMPOSITION COSMETIQUE COMPRENANT UN TENSIOACTIF, UN ALCOOL GRAS LIQUIDE ET UN ETHER D'ALCOOL GRAS OXYETHYLENE ET PROCÉDÉ DE TRAITEMENT COSMÉTIQUE**
KOSMETISCHE ZUSAMMENSETZUNG MIT EINEM TENSID, EINEM FLÜSSIGEN FETTALKOHOL UND EINEM OXYETHYLENIERTEM FETTALKOHOLETHER SOWIE KOSMETISCHES BEHANDLUNGSVERFAHREN DAMIT
COSMETIC COMPOSITION COMPRISING A SURFACTANT, A LIQUID FATTY ALCOHOL AND AN OXYETHYLENATED FATTY ALCOHOL ETHER AND COSMETIC TREATMENT METHOD

(30) Priorité: 17.12.2009 FR 0959123; 23.12.2009 US 289625 P
(43) Date de publication de la demande: 24.10.2012
(73) Titulaire: L'Oréal, 75008 Paris (FR)
(72) Inventeur: MATHONNEAU, Estelle, F-75010 Paris (FR)
(74) Mandataire: Dodin, Catherine
(86) Numéro de dépôt international: PCT/FR2010/052704
(87) Numéro de publication internationale: WO 2011/073563

(56) Documents cités:
- JP-A- 2009 073 932
- US-A- 5 437 809
- US-A- 5 656 200

## Description

La présente invention concerne une composition de lavage et de conditionnement des matières kératiniques, en particulier des fibres kératiniques, comprenant un tensioactif anionique, un alcool gras liquide et un éther de polyol oxyéthyléné, l'utilisation de ladite composition pour le lavage et le conditionnement des matières kératiniques, en particulier des fibres kératiniques, ainsi qu'un procédé de mise en oeuvre de ladite composition.

Dans le domaine des shampoings conditionneurs, on associe généralement une base lavante et un agent de conditionnement qui peut être un polymère cationique, un polymère amphotère, une silicone, une huile synthétique ou naturelle, un corps gras ou un de leurs mélanges. Ces agents de conditionnement sont utilisés pour améliorer le démêlage et la douceur des cheveux mouillés et séchés mais peuvent avoir tendance à alourdir la chevelure et à la ternir.

L'utilisation d'agents conditionneurs insolubles est fortement limitée :
- d'une part, du fait des difficultés de stabilisation dans des compositions détergentes, si on veut maintenir le niveau des qualités d'usage (stabilité, pouvoir moussant, démarrage de mousse),
- d'autre part, du fait des défauts cosmétiques en termes d'alourdissement, de charge et de regraissage liés à des dispersions grossières ou hétérogènes dans ces mêmes compositions détergentes.

Les agents de conditionnement insolubles, en particulier les alcools gras liquides, sont connus et utilisés dans les compositions de shampooings comme notamment dans les documents JP2002-20791, JP9-30938, US2009/005449 et US2009/005460.

Cependant, ces compositions ne sont pas épaissies et/ou stables et/ou ne présentent pas des performances cosmétiques de haut niveau en termes de démêlage et de lissage, et en maintenant le niveau des qualités d'usage.

En effet, on recherche des produits épaissis que l'on peut doser et prendre aisément dans la main. Pour ce faire, ces produits doivent présenter une certaine consistance ou viscosité. En effet, un produit liquide est beaucoup plus difficile à doser et s'écoule facilement entre les doigts.

Pour épaissir les compositions lavantes, on a déjà utilisé des polymères naturels tels que les celluloses. Malheureusement, ces épaississants donnent des compositions instables et/ou qui ne sont pas lisses et homogènes. De plus, ces épaississants présentent l'inconvénient de diminuer la qualité de la mousse et les performances cosmétiques des shampooings, notamment en rendant les cheveux plus chargés et plus rêches. La mousse des compositions épaissies n'est généralement pas suffisamment douce, elle ne se développe pas facilement que ce soit en vitesse ou en abondance.

Ainsi, il subsiste le besoin d'un système épaississant permettant d'épaissir, de manière convenable une composition lavante comprenant un alcool gras liquide sans influer sur les propriétés cosmétiques et moussantes desdites compositions ou en les améliorant.

La demanderesse a maintenant découvert que l'utilisation de d'éther d'alcool gras et de polyol oxyéthyléné particulier dans des compositions comprenant un tensioactif anionique et un alcool gras liquide permettait de surmonter les inconvénients indiqués ci-dessus, et d'obtenir ainsi une composition stable d'aspect homogène et esthétique. Par ailleurs, les propriétés cosmétiques et en particulier le lissage, la douceur, la souplesse et la brillance sont améliorés. Dans le cas des cheveux frisés et/ou crépus, on observe une diminution du volume permettant un meilleur contrôle de la chevelure.

Les compositions selon l'invention apportent également une protection de la couleur aux lavages des cheveux artificiellement colorés.

L'invention a donc pour objet une composition de lavage et de conditionnement des matières kératiniques, en particulier des fibres kératiniques, telle que définie à la revendication 1. Un autre objet de l'invention est l'utilisation de ladite composition pour le lavage et le conditionnement des matières kératiniques, en particulier des fibres kératiniques, et plus particulièrement des cheveux.

L'invention a encore pour objet un procédé de lavage et de conditionnement des fibres kératiniques mettant en oeuvre la composition selon l'invention.

D'autres objets, caractéristiques, aspects et avantages de l'invention apparaîtront encore plus clairement à la lecture de la description et des divers exemples qui suivent.

Dans la présente demande, on qualifie une entité comme étant "anionique" lorsqu'elle possède au moins une charge négative permanente ou lorsqu'elle peut être ionisée en une entité chargée négativement, dans les conditions d'utilisation des compositions de l'invention (milieu, pH par exemple) et ne comprenant pas de charge cationique.

On qualifie une entité comme étant "non ionique" lorsqu'elle n'est ni cationique ni anionique au sens de la présente demande, en particulier elle ne comporte aucun groupe cationique ou anionique au sens de la présente demande.

Par "cosmétiquement acceptable" ou "physiologiquement acceptable", on entend qui est compatible avec l'application sur le corps d'un être vivant, en particulier le corps humain, et notamment son cuir chevelu et ses cheveux.

Par composition épaissie on entend au sens de la présente invention une composition présentant une viscosité d'au moins 25 cps et de préférence d'au moins 50 cp à la température de 25°C et avec un taux de cisaillement de 1s⁻¹. Ces viscosités sont mesurables avec un viscosimètre ou un rhéomètre à géométrie cone-plan.

Les tensioactifs anioniques pouvant être utilisés dans la composition sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkylarylsulfonates, les alphaoléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-polycarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques poly-oxyalkylénés, les acides (alkyl en C₆₋₂₄)(aryl en C₆₋₂₄)éther-carboxyliques poly-oxyalkylénés, les acides (alkyl en C₆₋₂₄)amidoéther-carboxyliques poly-oxyalkylénés et leurs sels, en particulier ceux comportant de 2 à 50 motifs oxyde d'éthylène, et leurs mélanges.

On utilise de préférence les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

Le ou les tensioactifs anioniques sont de préférence présents en une quantité totale allant de 3 à 50 % en poids, mieux encore de 4 à 20% en poids par rapport au poids total de la composition.

Les« alcools gras liquides » sont liquides à température ambiante (25°C) et à pression atmosphérique, et insolubles dans l'eau (c'est-à-dire, présentent une solubilité dans l'eau inférieure à 1 % en poids et de préférence inférieure à 0,5 % en poids), et sont solubles, dans les mêmes conditions de température et de pression, dans au moins un solvant organique (par exemple l'éthanol, le chloroforme, le benzène ou l'huile de vaseline) à au moins 1 % en poids.

Les alcools gras liquides, en particulier ceux en C₁₀-C₃₀ présentent des chaînes carbonées ramifiées ou possèdent une ou plusieurs (de préférence 1 à 3) insaturations.

Les alcools gras liquides selon l'invention sont ramifiés et/ou insaturés, et présentent la structure R-OH, dans laquelle R désigne un groupement alkyle ramifié en C₁₂-C₂₄ ou alkényle en C₁₂-C₂₄. R peut être substitué par un ou plusieurs groupements hydroxy. De préférence, R ne contient pas de groupement hydroxy.

A titre d'exemple on peut citer l'alcool oléique, l'alcool linoléique, l'alcool linolénique, l'alcool isocétylique, l'alcool isostéarylique, le 2-octyl-1-dodécanol, le 2-butyl octanol, le 2-hexyl-1-décanol, le 2-decyl-1-tétradécanol, le 2-tétradécyl-1-cétanol et leur(s) mélange(s).

De préférence l'alcool gras liquide de l'invention est un alcool gras saturé ramifié. Encore plus préférentiellement l'alcool gras liquide de l'invention est le 2-octyl 1-dodécanol. Les alcools gras peuvent être des mélanges, ce qui signifie que dans un produit commercial peuvent coexister plusieurs espèces notamment de longueurs de chaînes différentes, sous forme d'un mélange.

Le ou les alcools gras liquides de l'invention sont généralement présents en une quantité allant de 0,01 à 10 % en poids, préférentiellement de 0,1 à 5% en poids et, mieux encore, de 0,5 à 3% en poids par rapport au poids total de la composition.

Les éthers d'alcool gras et de polyol oxyéthyléné présentent de préférence la formule (I) suivante :

R₁ (OCH₂CH₂)ₙ OR₂

n de 40 à 80.

R1 et R2 désignent un groupement alkyle linéaire ou ramifiée en C10-C30 et de préférence linéaire comportant éventuellement un groupement hydroxyle.

Un éther selon l'invention particulièrement préféré est le composé dénommé Ceteareth-60-Myristylglycol selon la dénomination INCI et notamment proposé sous le nom commercial d'ELFACOS GT282S par AKZO NOBEL.

Le ou les éthers d'alcools gras et de polyol oxyéthyléné sont généralement présents en une quantité allant de 0,01 à 10 % en poids, préférentiellement de 0,1 à 5% en poids et, mieux encore, de 0,5 à 3% en poids par rapport au poids total de la composition.

La composition peut également comprendre en outre au moins un tensioactif non ionique différent des éthers de l'invention de formule (I) et/ou au moins un tensioactif amphotère.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Parmi les tensioactifs non-ioniques cités ci-dessus, on utilise de préférence les (alkyl en C₆₋₂₄)polyglycosides, et plus particulièrement les (alkyl en C₈₋₁₈)polyglycosides.

Les agents tensioactifs amphotères, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₂₋₈)bétaïnes ou les (alkyl en C₈₋₂₀)amido(alkyl en C₂₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(C) (2)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle saturé ou insaturé en C7-C23, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères cités ci-dessus, on utilise de préférence les (alkyl en C₈₋₂₀)-bétaïnes, les (alkyl en C₈₋₂₀)-amido(alkyl en C₂₋₈)bétaïnes, les alkylamphodiacétates et leurs mélanges.

Les tensioactifs non ioniques et/ou amphotères sont de préférence présents dans la composition selon l'invention en une quantité allant de 0 à 20 % en poids, mieux encore de 0,5 à 10% en poids par rapport au poids total de la composition.

La quantité totale de tensioactifs, est de préférence comprise entre 4 et 50 % en poids, mieux encore entre 5 et 30 % en poids par rapport au poids total de la composition.

La composition selon l'invention peut notamment comprendre en outre un ou plusieurs polymères cationiques. Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 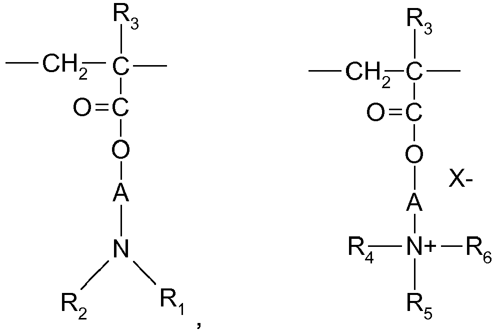 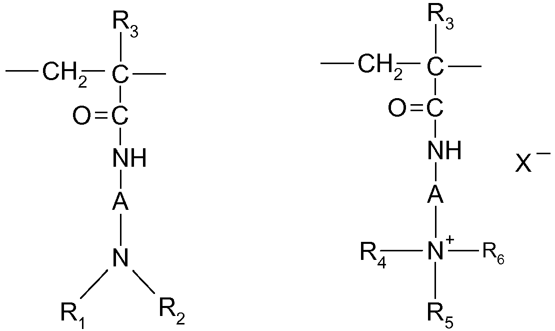 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyl-triméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyl-triméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP.
   - les copolymères vinylpyrrolidone / méthacrylamide de diméthylamino-propyle quaternisé tel que le produit vendu sous la dénomination "GAFQUAT HS 100" par la société ISP, et
   - les polymères de préférence réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (par exemple 20/80 en poids) en particulier sous forme de dispersion comprenant 50 % en poids dudit copolymère dans de l'huile minérale. Cette dispersion est commercialisée sous le nom de « SALCARE^{®} SC 92 » par la Société CIBA. On peut également utiliser un homopolymère réticulé du chlorure de méthacryloyloxyéthyl triméthylammonium et notamment comprenant environ 50 % en poids de l'homopolymère dans de l'huile minérale ou dans un ester liquide. Ces dispersions sont commercialisées sous les noms de « SALCARE^{®} SC 95 » et « SALCARE^{®} SC 96 » par la Société CIBA.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US 3 589 578 et 4 031 307 en particulier les gommes de guar comprenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (I) ou (II) : 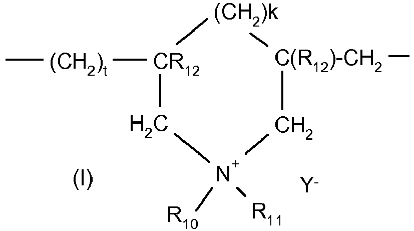 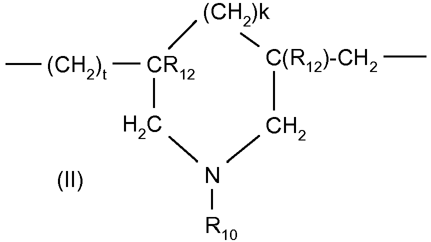 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de sels (par exemple chlorure) de diméthyldiallylammonium vendu sous la dénomination "MERQUAT 100" par la société NALCO (et leurs homologues de faibles masses molaires moyenne en poids) et les copolymères de sels (par exemple chlorure) de diallyldiméthylammonium et d'acrylamide commercialisés sous la dénomination "MERQUAT 550".
(8) les polymères de diammonium quaternaire comprenant des motifs récurrents répondant à la formule : 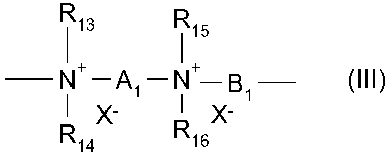 formule (III) dans laquelle :
   R₁₃, R₁₄, R₁₅ et R₁₆, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou - CO-NH-R₁₇-D où R₁₇ est un alkylène et D un groupement ammonium quaternaire ;
   A1 et B1 représentent des groupements polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- ;

   De préférence, X- est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 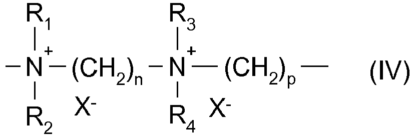 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (IV) particulièrement préféré est celui pour lequel R₁, R₂, R₃ et R₄, représentent un radical méthyle et n = 3, p = 6 et X = Cl, dénommé Hexadimethrine chloride selon la nomenclature INCl (CTFA).
(9) les polymères de polyammonium quaternaires comprenant des motifs de formule (V): 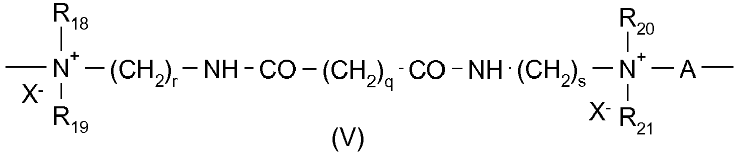 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou représente de préférence

      -CH₂-CH₂-O-CH₂-CH₂-.

   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de « POLYETHYLENEGLYCOL (15) TALLOW POLYAMINE » dans le dictionnaire CTFA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires tels que les produits vendus sous la dénomination « JR 400 » par la Société AMERCHOL, les cyclopolymères cationiques, en particulier les homopolymères ou copolymères de sels (par exemple chlorure) de diméthyldiallylammonium, vendus sous les dénominations « MERQUAT 100 », « MERQUAT 550 » et « MERQUAT S » par la société NALCO et leurs homologues de faibles poids moléculaires en poids, les polymères quaternaires de vinylpyrrolidone et de vinylimidazole, les homopolymères ou copolymères éventuellement réticulés de sels de méthacryloyloxyalkyl(C₁-C₄) trialkyl(C₁-C₄)ammonium et leurs mélanges.

Le ou les polymères cationiques sont généralement présents à des concentrations allant de 0,01 à 20%, de préférence de 0,05 à 10% et plus particulièrement de 0,1 à 5 % en poids par rapport au poids total de la composition.

La composition selon l'invention peut notamment comprendre en outre au moins une silicone. Cette silicone peut être linéaire, ramifiée ou cyclique, volatile ou non, organomodifiée ou non.

Par milieu cosmétiquement acceptable, on entend un milieu compatible avec les matières kératiniques, notamment les fibres kératiniques telles que les cheveux, mais aussi d'odeur, d'aspect et de toucher agréables. Le milieu est de préférence aqueux.

Le milieu cosmétiquement acceptable est constitué d'eau ou d'un mélange d'eau et d'au moins un solvant organique choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols.

De préférence, la composition comprend de 70 à 95 % en poids d'eau par rapport au poids total de la composition.

Le pH des compositions selon l'invention est généralement compris entre 2 et 11, de préférence entre 3 et 10 et, mieux encore entre 4 et 8.

La composition selon l'invention peut comprendre en outre des additifs choisis parmi les polymères anioniques, les polymères non ioniques, ou les polymères amphotères, les épaississants polymères associatifs ou non, les épaississants non polymères, les agents nacrants, les opacifiants, les colorants ou les pigments, les parfums, les huiles minérales, végétales ou synthétiques, les cires, les vitamines, les filtres UV, les agents anti-radicalaires, les antipelliculaires, les conservateurs, les agents de stabilisation de pH, les solvants, et leurs mélanges.

L'homme du métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions peuvent être conditionnées sous diverses formes notamment dans des flacons, des flacons-pompe ou dans des récipients aérosols afin d'assurer une application de la composition sous forme vaporisée ou sous forme de mousse. Les compositions peuvent également imprégner des applicateurs, notamment des gants ou des lingettes.

La présente invention concerne également un procédé de lavage des matières kératiniques qui consiste à appliquer une quantité efficace d'une composition telle que décrite ci-dessus, sur les matières kératiniques, à effectuer un rinçage par exemple avec de l'eau après un éventuel temps de pose.

Les exemples suivants sont donnés à titre illustratif de la présente invention. Toutes les quantités indiquées sont exprimées en % en poids, sauf indication contraire.

### Exemples 1

On a préparé la composition de shampooing suivante :

| Composition | Invention |
|---|---|
| Lauryléthersulfate de sodium (C12/C14 à 70/30) à 2.2 moles d'oxyde d'éthylène (TEXAPON AOS 225 UP de COGNIS) | 4,9 g MA |
| Laurylsulfate de sodium (C12/C14 à 70/30) (TEXAPON LS 35 de COGNIS) | 2,6 gMA |
| Cocoamidopropyl bétaïne (DEHYTON PK 45 de COGNIS) | 3,05 g MA |
| 2-octyl dodécanol | 2 g |
| Alcool cétylstéarylique (C16/C18) oxyéthyléné (60 OE) éther de myristyl glycol (ELFACOS GT 282 S de AKZO NOBEL) | 2 g |
| Polyquaternium-10 (POLYMER JR400 LT de AMERCHOL) | 0,4 g |
| Chlorure de sodium | 0,5 g |
| Glycérine | 0,5 g |
| Conservateurs, parfum | q,s, |
| Acide citrique q.s. | pH 5,3 |
| Eau déminéralisée q.s.p. | 100 g |

| | |
|---|---|
| * : en Matière active (MA) | |

La composition conforme à l'invention est épaissie et présente un aspect homogène et lisse, contrairement à une composition comparative ne comprenant pas l'ELFACOS GT 282S qui est totalement fluide.

Les cheveux traités avec la composition de l'exemple 1 sont lisses et faciles à démêler.

### EXEMPLES 2 et 3

On a préparé les compositions de shampooing suivantes :

| Composition | Exemple 2 | Exemple 3 |
|---|---|---|
| Lauryléthersulfate de sodium (C12/C14 à 70/30) à 2.2 moles d'oxyde d'éthylène (TEXAPON AOS 225 UP de COGNIS) | 7,7 g MA | 4,9 g MA |
| Laurylsulfate de sodium (C12/C14 à 70/30) (TEXAPON LS 35 de COGNIS) | 2,6 g MA | 4 g MA |
| Disodium cocoamphodiacétate (MIRANOL C2M CONC NP de RHODIA) | 2,4 gMA | 1,3 gMA |
| Hydroxyéthylcellulose réticulée par l'épichlorhydrine et quaternisée par la triméthylamine (UCARE POLYMER JR 400 LT par la société AMERCHOL) | 0,4 g | 0,4 g |
| 2-octyl 1-dodécanol | 1,5 g | 2 g |
| Alcool cétylstéarylique (C16/C18) oxyéthyléné (60 OE) éther de myristyl glycol (ELFACOS GT 282 S de AKZO NOBEL) | 1,5 g | 2 g |
| Conservateurs, parfum | q.s. | q.s. |
| Acide citrique q.s. | pH 6,5 | pH 6,3 |
| Eau déminéralisée q.s.p. | 100 g | 100 g |

Les cheveux traités avec les compositions des exemples 2 ou 3 sont lisses.

Autre composition selon l'invention :

### EXEMPLE 4

| Composition | Exemple 4 |
|---|---|
| Lauryléthersulfate de sodium (C12/C14 à 70/30) à 2.2 moles d'oxyde d'éthylène (TEXAPON AOS 225 UP de COGNIS) | 7,7 g MA |
| Laurylsulfate de sodium (C12/C14 à 70/30) (TEXAPON LS 35 de COGNIS) | 2,6 g MA |
| Disodium cocoamphodiacétate (MIRANOL C2M CONC NP de RHODIA) | 2,4 gMA |
| Hydroxyéthylcellulose réticulée par l'épichlorhydrine et quaternisée par la triméthylamine (UCARE POLYMER JR 400 LT par la société AMERCHOL) | 0,4 g |
| Alcool oléique | 1,5 g |
| Alcool cétylstéarylique (C16/C18) oxyéthyléné (60 OE) éther de myristyl glycol (ELFACOS GT 282 S de AKZO NOBEL) | 2 g |
| Conservateurs, parfum | q.s. |
| Acide citrique q.s. | pH 6,5 |
| Eau déminéralisée q.s.p. | 100 g |

### EXEMPLE 5

On a préparé la composition de shampooing suivante :

| Composition | Exemple 5 |
|---|---|
| Lauryléthersulfate de sodium (C12/C14 à 70/30) à 2.2 moles d'oxyde d'éthylène (TEXAPON AOS 225 UP de COGNIS) | 4,9 g MA |
| Laurylsulfate de sodium (C12/C14 à 70/30) (TEXAPON LS 35 de COGNIS) | 2,6 g MA |
| Disodium cocoamphodiacétate (MIRANOL C2M CONC NP de RHODIA) | 2,4 gMA |
| Hydroxyéthylcellulose réticulée par l'épichlorhydrine et quaternisée par la triméthylamine (UCARE POLYMER JR 400 LT par la société AMERCHOL) | 0,4 g |
| 2 décyl-1-tétradécanol | 1,5 g |
| Alcool cétylstéarylique (C16/C18) oxyéthyléné (60 OE) éther de myristyl glycol (ELFACOS GT 282 S de AKZO NOBEL) | 2 g |
| Conservateurs, parfum | q.s. |
| Acide citrique q.s. | pH 6,3 |
| Eau déminéralisée q.s.p. | 100 g |

### EXEMPLES 6 et 7

On a préparé les compositions de shampooing suivantes :

| Composition | Exemple 6 | Exemple 7 |
|---|---|---|
| Lauryléthersulfate de sodium (C12/C14 à 70/30) à 2.2 moles d'oxyde d'éthylène (TEXAPON AOS 225 UP de COGNIS) | 5,25 g MA | 5,25 g MA |
| Laurylsulfate de sodium (C12/C14 à 70/30) (TEXAPON LS 35 de COGNIS) | 4 g MA | 4 g MA |
| Disodium cocoamphodiacétate (MIRANOL C2M CONC NP de RHODIA) | 1,2 gMA | 1,2 gMA |
| Chlorure d'hydroxypropyl guar triméthylammonium (JAGUAR C162 de RHODIA) | 0,15 g | 0,15 g |
| 2 décyl-1-tétradécanol | 0,05 g | |
| 2-octyl 1-dodécanol | | 1,5 g |
| Alcool cétylstéarylique (C16/C18) oxyéthyléné (60 OE) éther de myristyl glycol (ELFACOS GT 282 S de AKZO NOBEL) | 3 g | 3 g |
| Chlorure de sodium | 0,5 g | 0,5 g |
| Conservateurs, parfum | q.s. | q.s. |
| Acide citrique q.s. | pH 6,5 | pH 6,3 |
| Eau déminéralisée q.s.p. | 100 g | 100 g |

## Revendications

1. Composition de lavage des matières kératiniques, **caractérisée par le fait qu'**elle comprend, dans un milieu cosmétiquement acceptable :
- un ou plusieurs tensioactifs anioniques,
- un ou plusieurs alcools gras ramifiés et/ou insaturés, liquides à température ambiante (25°C) et à pression atmosphérique, présentant la structure R-OH, dans laquelle R désigne un groupement alkyle ramifié en C12-C24 ou alkényle en C12-C24; et
- un ou plusieurs éthers d'alcool gras et de polyol oxyéthyléné de formule (I) suivante :
R₁(OCH₂CH₂)ₙOR₂
dans laquelle :
- R1 désigne un groupement alkyle linéaire ou ramifié en C10-C30, éventuellement substitué par un groupement hydroxyle,
- R2 désigne un groupement alkyle linéaire ou ramifié en C10-C30 éventuellement substitué par un groupement hydroxyle, et
- n va de 40 à 80.

2. Composition selon la revendication précédente, **caractérisée en ce que** le tensioactif anionique est choisi parmi les alkylsulfates, les alkyléthersulfates et les alkyléthercarboxylates, et leurs mélanges.

3. Composition selon l'une des revendications précédentes, **caractérisée en ce que** le ou les tensioactifs anioniques sont présents en une quantité allant de 3 à 50% en poids par rapport au poids total de la composition.

4. Composition selon l'une des revendications précédentes, **caractérisée en ce que** les alcools gras sont choisis parmi l'alcool oléique, l'alcool isocétylique, l'alcool isostéarylique, le 2-octyl-1-dodécanol, le 2-butyl octanol, le 2-hexyl-1-décanol, le 2-decyl-1-tétradécanol, le 2-tétradécyl-1-cétanol et leur(s) mélange(s).

5. Composition selon l'une des revendications précédentes, **caractérisée en ce que** le ou les alcools gras liquides sont présents en une quantité allant de 0,01 à 10% en poids par rapport au poids total de la composition.

6. Composition selon l'une des revendications précédentes, **caractérisée en ce que** le ou les éthers d'alcools gras et de polyol oxyéthyléné sont présents en une quantité allant de 0,01 à 10% en poids, préférentiellement de 0,1 à 5% en poids et, mieux encore de 0,5 à 3% en poids par rapport au poids total de la composition.

7. Composition selon l'une des revendications précédentes, **caractérisée en ce qu'**elle comprend en outre au moins un tensioactif non ionique différent des éthers de formule (I) et/ou au moins un tensioactif amphotère.

8. Composition selon la revendication 7, **caractérisée en ce que** le ou les tensioactifs non ioniques et/ou amphotères sont présents en une quantité totale allant de 0 à 20% en poids, de préférence allant de 0,5 à 20% en poids par rapport au poids total de la composition.

9. Composition selon l'une des revendications précédentes, **caractérisée en ce que** le milieu cosmétiquement acceptable est constitué d'eau, ou d'un mélange d'eau et d'au moins un solvant organique.

10. Composition selon l'une des revendications précédentes, **caractérisée en ce qu'**elle comprend d'autres ingrédients choisis parmi les silicones, les polymères anioniques, les polymères cationiques, les polymères non ioniques ou les polymères amphotères, les épaississants polymères associatifs ou non, les épaississants non polymères, les agents nacrants, les opacifiants, les colorants ou les pigments, les parfums, les huiles minérales, végétales ou synthétiques, les cires, les vitamines, les filtres UV, les agents anti-radicalaires, les antipelliculaires, les conservateurs, les agents de stabilisation de pH, les solvants, et leurs mélanges.

11. Utilisation de la composition selon l'une des revendications précédentes, pour le lavage et le conditionnement des matières kératiniques et en particulier des fibres kératiniques.

12. Procédé de lavage et de conditionnement des matières kératiniques et en particulier des fibres kératiniques, comprenant l'application d'une quantité efficace d'une composition selon l'une quelconque des revendications 1 à 10, sur les cheveux, et un rinçage après un éventuel temps de pose.

## Patentansprüche

1. Zusammensetzung zum Waschen von Keratinmaterialien, **dadurch gekennzeichnet, dass** sie in einem kosmetisch unbedenklichen Medium Folgendes umfasst:
- ein oder mehrere anionische Tenside,
- einen oder mehrere verzweigte und/oder ungesättigte, bei Umgebungstemperatur (25 °C) und Normaldruck flüssige Fettalkohole mit der Struktur R-OH, wobei R für eine verzweigte C12-C24-Alkyl- oder C12-C24-Alkenylgruppe steht; und
- einen oder mehrere Ether eines Fettalkohols und eines oxyethylenierten Polyols der folgenden Formel (I):
R₁(OCH₂CH₂)ₙOR₂,
in der:
- R1 für eine lineare oder verzweigte C10-C30-Alkylgruppe, die gegebenenfalls durch eine Hydroxylgruppe substituiert ist, steht,
- R2 für eine lineare oder verzweigte C10-C30-Alkylgruppe, die gegebenenfalls durch eine Hydroxylgruppe substituiert ist, steht und
- n im Bereich von 40 bis 80 liegt.

2. Zusammensetzung nach dem vorhergehenden Anspruch, **dadurch gekennzeichnet, dass** das anionische Tensid aus Alkylsulfaten, Alkylethersulfaten und Alkylethercarboxylaten und Mischungen davon ausgewählt ist.

3. Zusammensetzung nach einem der vorhergehenden Ansprüche, **dadurch gekennzeichnet, dass** das anionische Tensid bzw. die anionischen Tenside in einer Menge im Bereich von 3 bis 50 Gew.-%, bezogen auf das Gesamtgewicht der Zusammensetzung, vorliegt bzw. vorliegen.

4. Zusammensetzung nach einem der vorhergehenden Ansprüche, **dadurch gekennzeichnet, dass** die Fettalkohole aus Oleylalkohol, Isocetylalkohol, Isostearylalkohol, 2-Octyl-1-dodecanol, 2-Butyloctanol, 2-Hexyl-1-decanol, 2-Decyl-1-tetra-decanol, 2-Tetradecyl-1-cetanol und Mischungen davon ausgewählt sind.

5. Zusammensetzung nach einem der vorhergehenden Ansprüche, **dadurch gekennzeichnet, dass** der flüssige Fettalkohol bzw. die flüssigen Fettalkohole in einer Menge im Bereich von 0,01 bis 10 Gew.-%, bezogen auf das Gesamtgewicht der Zusammensetzung, vorliegt bzw. vorliegen.

6. Zusammensetzung nach einem der vorhergehenden Ansprüche, **dadurch gekennzeichnet, dass** der Ether bzw. die Ether eines Fettalkohols und eines oxyethylenierten Polyols in einer Menge im Bereich von 0,01 bis 10 Gew.-%, vorzugsweise von 0,1 bis 5 Gew.-% und noch besser von 0,5 bis 3 Gew.-%, bezogen auf das Gesamtgewicht der Zusammensetzung, vorliegt bzw. vorliegen.

7. Zusammensetzung nach einem der vorhergehenden Ansprüche, **dadurch gekennzeichnet, dass** sie außerdem mindestens ein nichtionisches Tensid, das von den Ethern der Formel (I) verschieden ist, und/oder mindestens ein amphoteres Tensid umfasst.

8. Zusammensetzung nach Anspruch 7, **dadurch gekennzeichnet, dass** das nichtionische und/oder amphotere Tensid bzw. die nichtionischen und/oder amphoteren Tenside in einer Gesamtmenge im Bereich von 0 bis 20 Gew.-%, vorzugsweise im Bereich von 0,5 bis 20 Gew.-%, bezogen auf das Gesamtgewicht der Zusammensetzung, vorliegen.

9. Zusammensetzung nach einem der vorhergehenden Ansprüche, **dadurch gekennzeichnet, dass** das kosmetisch unbedenkliche Medium aus Wasser oder einer Mischung von Wasser und mindestens einem organischen Lösungsmittel besteht.

10. Zusammensetzung nach einem der vorhergehenden Ansprüche, **dadurch gekennzeichnet, dass** sie weitere Bestandteile umfasst, die aus Silikonen, anionischen Polymeren, kationischen Polymeren, nichtionischen Polymeren oder amphoteren Polymeren, assoziativen oder nichtassoziativen polymeren Verdickern, nichtpolymeren Verdickern, Perlglanzmitteln, Trübungsmitteln, Farbstoffen oder Pigmenten, Duftstoffen, mineralischen, pflanzlichen oder synthetischen Ölen, Wachsen, Vitaminen, UV-Schutzmitteln, Radikalfängern, Antischuppenmitteln, Konservierungsstoffen, pH-Stabilisatoren, Lösungsmitteln und Mischungen davon ausgewählt sind.

11. Verwendung der Zusammensetzung nach einem der vorhergehenden Ansprüche zum Waschen und Konditionieren von Keratinmaterialien und insbesondere Keratinfasern.

12. Verfahren zum Waschen und Konditionieren von Keratinmaterialien und insbesondere Keratinfasern, bei dem man eine wirksame Menge einer Zusammensetzung nach einem der Ansprüche 1 bis 10 auf die Haare aufbringt und nach einer fakultativen Einwirkungszeit spült.

## Claims

1. Composition for washing keratin materials, **characterized in that** it comprises, in a cosmetically acceptable medium:
- one or more anionic surfactants,
- one or more branched and/or unsaturated fatty alcohols, having the structure R-OH, in which R denotes a C₁₂-C₂₄ branched alkyl or C₁₂-C₂₄ alkenyl group; and
- one or more ethers of a fatty alcohol and of an oxyethylenated polyol of formula (I) below:
R₁ (OCH₂CH₂)ₙ OR₂
in which:
- R₁ denotes a linear or branched C₁₀-C₃₀ alkyl group, optionally substituted with a hydroxyl group,
- R₂ denotes a linear or branched C₁₀-C₃₀ alkyl group optionally substituted with a hydroxyl group, and
- n ranges from 40 to 80.

2. Composition according to the preceding claim, **characterized in that** the anionic surfactant is chosen from alkyl sulfates, alkyl ether sulfates and alkyl ether carboxylates, and mixtures thereof.

3. Composition according to either of the preceding claims, **characterized in that** the anionic surfactant(s) are present in an amount ranging from 3% to 50% by weight relative to the total weight of the composition.

4. Composition according to one of the preceding claims, **characterized in that** the fatty alcohols are chosen from oleyl alcohol, isocetyl alcohol, isostearyl alcohol, 2-octyl-1-decanol, 2-butyloctanol, 2-hexyl-1-decanol, 2-decyl-1-tetradecanol and 2-tetradecyl-1-cetanol, and mixtures thereof.

5. Composition according to one of the preceding claims, **characterized in that** the liquid fatty alcohol(s) are present in an amount ranging from 0.01% to 10% by weight relative to the total weight of the composition.

6. Composition according to one of the preceding claims, **characterized in that** the ether(s) of fatty alcohols and of oxyethylenated polyols are present in an amount ranging from 0.01% to 10% by weight, preferentially from 0.1% to 5% by weight and better still from 0.5% to 3% by weight relative to the total weight of the composition.

7. Composition according to one of the preceding claims, **characterized in that** it also comprises at least one nonionic surfactant other than the ethers of formula (I) and/or at least one amphoteric surfactant.

8. Composition according to Claim 7, **characterized in that** the nonionic and/or amphoteric surfactant(s) are present in a total amount ranging from 0 to 20% by weight and preferably from 0.5% to 20% by weight relative to the total weight of the composition.

9. Composition according to one of the preceding claims, **characterized in that** the cosmetically acceptable medium consists of water or of a mixture of water and of at least one organic solvent.

10. Composition according to one of the preceding claims, **characterized in that** it comprises other ingredients chosen from silicones, anionic polymers, cationic polymers, nonionic polymers, amphoteric polymers, associative or non-associative polymeric thickeners, non-polymeric thickeners, nacreous agents, opacifiers, dyes or pigments, fragrances, mineral, plant or synthetic oils, waxes, vitamins, UV-screening agents, free-radical scavengers, antidandruff agents, preserving agents, pH stabilizers and solvents, and mixtures thereof.

11. Use of the composition according to one of the preceding claims, for washing and conditioning keratin materials and in particular keratin fibres.

12. Process for washing and conditioning keratin materials and in particular keratin fibres, comprising the application of an effective amount of a composition according to any one of Claims 1 to 10 to the hair, and rinsing after an optional leave-on time.
